# EUROPEAN PATENT APPLICATION

(11) **EP 4 644 882 A1**
(43) Date of publication of application: **05.11.2025**
(21) Application number: 23910405.2
(22) Date of filing: 22.12.2023
(51) Int. Cl.: G01N 23/04, G01N 33/12

(54) **MEAT INSPECTION SYSTEM AND MEAT INSPECTION METHOD**

(30) Priority: 27.12.2022 CN 202211714433
(71) Applicant: Tsinghua University, Haidian District, Beijing 100084 (CN); NUCTECH COMPANY LIMITED, Beijing 100084 (CN)
(72) Inventor: CHEN, Zhiqiang, Beijing 100084 (CN); ZHANG, Li, Beijing 100084 (CN); SUN, Yunda, Beijing 100084 (CN); LI, Xinbin, Beijing 100084 (CN); SHEN, Le, Beijing 100084 (CN); ZHAO, Zhenhua, Beijing 100084 (CN)
(74) Representative: Gunzelmann, Rainer
(86) International application number: PCT/CN2023/141084
(87) International publication number: WO 2024/140479

(57) **Abstract**

A meat inspection system (1000) and a meat inspection method. The system (1000) comprises: a conveying device (10) defining a conveying channel (110) for conveying a meat product (20), wherein the meat product (20) moves in a conveying direction (D) on the conveying channel (110); a ray source (30) configured to emit a ray beam (R) towards the conveying channel (110), wherein the ray beam (R) crosses the conveying channel (110) in a transverse direction (L) relative to the conveying direction (D); a detector (40) configured to receive the ray beam (R) to respectively generate a first detection signal and a second detection signal, wherein the first detection signal and the second detection signal correspond to radiation of different energy ranges; and a processor (60) configured to generate a first transmission image and a second transmission image on the basis of the first detection signal and the second detection signal detected by the detector (40), and generate a transmission scanning image of the meat product (20) on the basis of the first transmission image and the second transmission image, thereby calculating a quantitative indicator of the meat product (20) so as to grade the meat product (20).

## Description

This application claims priority to Chinese Patent Application No. 202211714433.4, filed on 27 December 2022, the entire disclosure of which is incorporated herein by reference.

### Technical Field

The present disclosure relates to the field of inspection technology. Specifically, it relates to a meat inspection system and a meat inspection method, particularly a meat inspection system and method based on ray imaging.

### Background

In slaughterhouses and meat processing plants for pork, lamb, etc., it is necessary to grade meat products to settle payments with suppliers and determine factory prices based on grading results. Taking pork as an example, current domestic pig slaughterhouses primarily measure backfat thickness to manually grade pork products in combination with visual observation. Since backfat thickness is only a local characteristic of the meat and cannot represent the overall lean-to-fat ratio, and visual observation heavily depends on the experience and integrity of the grading personnel, the grading results may have significant errors, potentially causing economic losses to slaughterhouses.

Currently, ultrasonic inspection methods are more popular among foreign manufacturers. However, several issues arise when introducing this technology domestically: First, the associated equipment is prohibitively expensive, making it difficult for slaughterhouses to recoup costs within a few years, rendering it economically unviable. Secondly, the pigs slaughtered by foreign manufacturers are more uniform in breed, size, and weight, whereas domestic pigs come from diverse sources (small-scale or centralized farming), with significant variations in breed, size, and weight.

For accurate pork grading, a more complex database is needed, which would require excessive time and effort, making it impractical.

### Summary

According to an aspect of the present disclosure, a meat inspection system is provided. The meat inspection system comprises: a conveying device defining a conveying channel for conveying a meat product, the meat product moving along a conveying direction on the conveying channel; a ray source configured to emit a ray beam toward the conveying channel, the ray beam crossing the conveying channel along a transverse direction relative to the conveying direction; a detector configured to receive the ray beam to generate a first detection signal and a second detection signal respectively, wherein the first detection signal and the second detection signal correspond to radiations of different energy ranges; and a processor configured to generate a first transmission image and a second transmission image based on the first detection signal and the second detection signal, generate a transmission scan image of the meat product by combining the first transmission image and the second transmission image, and calculate a quantitative indicator of the meat product based on the transmission scan image to grade the meat product.

Optionally, the meat inspection system further comprises a three-dimensional imaging device configured to obtain three-dimensional contour information of the meat product, the processor calculates the quantitative indicator based on the three-dimensional contour information, the first transmission image and the second transmission image.

Optionally, the ray beam emitted from the ray source is a single ray beam, and the detector is configured to include a first energy detector and a second energy detector, the first energy detector receives a first energy component of the single ray beam to generate the first detection signal, and the second energy detector receives a second energy component of the single ray beam to generate the second detection signal.

Optionally, the ray beam emitted from the ray source includes a first energy ray beam and a second energy ray beam with different energy ranges, and the detector is configured to receive the first energy ray beam and the second energy ray beam to generate the first detection signal and the second detection signal, respectively.

Optionally, the ray source is configured to alternately emit the first energy ray beam and the second energy ray beam.

Optionally, the ray source is configured to include a first energy ray source and a second energy ray source, the first energy ray source emits the first energy ray beam, and the second energy ray source emits the second energy ray beam.

Optionally, the system is configured to include one or multiple ray sources, the multiple ray sources are arranged along the transverse direction, such that ray beams sequentially emitted from one side of the conveying channel to the other side of the conveying channel cover a transverse width of the conveying channel.

Optionally, the detector is further configured to generate at least one additional detection signal, wherein the additional detection signal is different from the first detection signal and the second detection signal.

Optionally, an energy range corresponding to the additional detection signal is different from energy ranges corresponding to the first detection signal and the second detection signal.

Optionally, the processor is further configured to classify parts of the meat product based on the three-dimensional contour information and calculate quantitative indicators for different parts of the meat product based on the transmission scanning image, thereby grading the different parts of the meat product.

Optionally, the three-dimensional imaging device includes at least one selected from a group consisting of a 3D camera, a visible light camera, an infrared camera and a LiDAR camera.

Optionally, the conveying device comprises an overhead rail device configured to move the meat product with the meat product being suspended; or a conveyor belt configured to allow the meat product to be placed thereon for conveying the meat product.

Optionally, the meat inspection system further includes a posture adjustment device to adjust a posture of the meat product to be loaded onto the conveying channel in coordination with the conveying device.

Optionally, the quantitative indicator includes at least one selected from a group consisting of lean meat percentage, length information, width information, weight information and inclusions.

An aspect of the disclosure provides a meat inspection method, comprising: conveying a meat product by a conveying device, wherein the conveying device defines a conveying channel for conveying the meat product, and the meat product moves along a conveying direction on the conveying channel; emitting a ray beam toward the conveying channel by a ray source, wherein the ray beam crosses the conveying channel along a transverse direction relative to the conveying direction; receiving the ray beam by a detector to generate a first detection signal and a second detection signal respectively, wherein the first detection signal and the second detection signal correspond to radiations of different energy ranges; generating a first transmission image and a second transmission image by a processor based on the first detection signal and the second detection signal, and generating a transmission scan image of the meat product by combining the first transmission image and the second transmission image, thereby calculating a quantitative indicator of the meat product to grade the meat product.

Optionally, the method further comprises: obtaining three-dimensional contour information of the meat product by a three-dimensional imaging device. The processor calculates the quantitative indicator based on the three-dimensional contour information, the first transmission image and the second transmission image.

Optionally, the ray beam emitted from the ray source is a single ray beam, and the detector is configured to include a first energy detector and a second energy detector, the first energy detector receives a first energy component of the single ray beam to generate the first detection signal, and the second energy detector receives a second energy component of the single ray beam to generate the second detection signal.

Optionally, the ray beam emitted from the ray source includes a first energy ray beam and a second energy ray beam with different energy ranges, and the detector is configured to receive the first energy ray beam and the second energy ray beam to generate the first detection signal and the second detection signal, respectively.

Optionally, the ray source is configured to alternately emit the first energy ray beam and the second energy ray beam.

Optionally, the ray source is configured to include a first energy ray source and a second energy ray source, wherein the first energy ray source emits the first energy ray beam, and the second energy ray source emits the second energy ray beam.

Optionally, the ray beam is emitted by one or multiple ray sources, the multiple ray sources are arranged along the transverse direction, such that ray beams sequentially emitted from one side of the conveying channel to the other side of the conveying channel cover a transverse width of the conveying channel.

Optionally, the detector is further configured to generate at least one additional detection signal, wherein the additional detection signal is different from the first detection signal and the second detection signal.

Optionally, the processor classifies parts of the meat product based on the three-dimensional contour information and calculate quantitative indicators for different parts of the meat product based on the transmission scanning image, thereby grading the different parts of the meat product.

Optionally, the three-dimensional imaging device includes at least one selected from a group consisting of a 3D camera, a visible light camera, an infrared camera and a LiDAR camera.

Optionally, the conveying device comprises: an overhead rail device configured to move the meat product with the meat product being suspended; or a conveyor belt configured to allow the meat product to be placed thereon for conveying the meat product.

Optionally, the method further comprises: adjusting a posture of the meat product to be loaded onto the conveying channel by a posture adjustment device in coordination with the conveying device.

Optionally, the quantitative indicator includes at least one selected from a group consisting of lean meat percentage, length information, width information, weight information and inclusions.

It should be understood that the descriptions herein are not intended to identify key or essential features of the embodiments of the disclosure, nor to limit the scope of the disclosure. Other features of the present disclosure will become apparent from the following description.

### Brief Description of Drawings

The drawings are provided to facilitate understanding of the solution and do not constitute a limitation of the disclosure, in which:
Figure 1 shows a schematic diagram of a meat inspection system according to an embodiment of the present disclosure.
Figures 2A, 2B, and 2C respectively show schematic diagrams of different embodiments of the meat inspection system irradiating a meat product along line C-C in Figure 1.
Figure 3A shows curves of mass attenuation coefficients (µ/ρ) of three types of tissues as a function of X-ray energy.
Figure 3B shows curves of linear attenuation coefficients (µ) of three types of tissues as a function of X-ray energy.
Figure 4 shows a schematic diagram of three-dimensional imaging of a meat product according to an embodiment of the present disclosure.
Figure 5 shows a data processing block diagram of the meat inspection system according to an embodiment of the present disclosure.
Figure 6A shows a schematic diagram of the meat inspection system according to an embodiment of the present disclosure.
Figure 6B shows a schematic diagram of the meat inspection system irradiating a meat product along line C-C in Figure 6A.
Figure 7 shows the arrangement of a distributed ray source in a meat detection system according to an embodiment of the present disclosure.
Figure 8 shows an overhead rail device according to an embodiment of the present disclosure.
Figure 9 shows a conveyor belt according to an embodiment of the present disclosure.

### Detailed Description of Embodiments

To clearly explain the objectives, technical solutions, and advantages of the present disclosure, embodiments of the disclosure will be described in detail with reference to the accompanying drawings. It should be understood that the described embodiments are intended to explain the general concept of the disclosure and should not be construed as limitations to the application. In the description and drawings, the same or similar reference numerals denote the same or similar components or elements. For clarity, the drawings may not be drawn to scale, and some well-known components or structures may be omitted.

Unless otherwise defined, technical or scientific terms used herein shall have the meanings commonly understood by those skilled in the art. Terms such as "first," "second," and similar expressions used herein do not denote any order, quantity, or importance but are merely used to distinguish different components. The wordings "a" or "an" do not exclude plurality. Terms such as "comprising" or "including" mean that the elements or items preceding the term encompass the elements or items listed thereafter and their equivalents, but do not exclude other elements or items. Terms such as "connected" or "coupled" are not limited to physical or mechanical connections but may include electrical connections, whether direct or indirect. Terms such as "upper," "lower," "left," "right," "top," or "bottom" are used to describe relative positional relationships, and these relationships may change if an absolute position of the described object changes. In case an element such as a layer, film, region, or substrate is described as being "above" or "below" another element, it may be directly "on" or "under" the another element, or there may be intermediate elements between them.

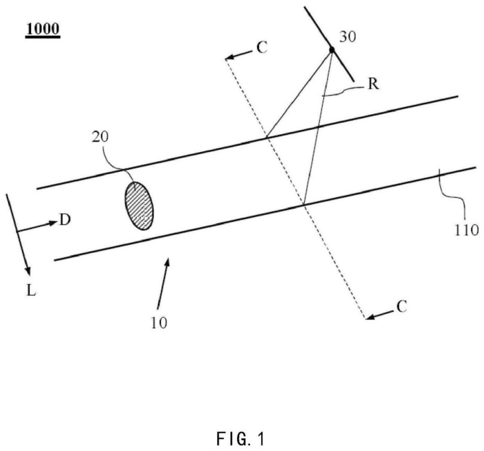

Figure 1 shows a schematic diagram of a meat inspection system 1000 according to an embodiment of the present disclosure.

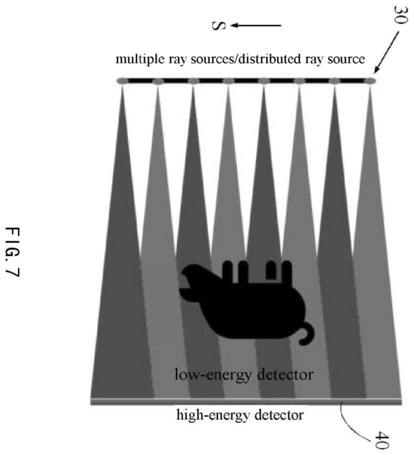

As shown in Figure 1, a meat inspection system 1000 includes a conveying device 10. The conveying device 10 is used to convey a meat product 20 along a conveying direction D. According to an embodiment of the disclosure, the conveying device 10 may be a device used in the production line of a meat slaughterhouse to convey meat products 20 for processing. For example, the conveying device 10 may be a conveying device being currently used in slaughterhouse production lines. As shown in Figure 1, the conveying device 10 defines a conveying channel 110 for conveying the meat product 20. The term "conveying channel 110" herein may be understood as a channel formed by the space occupied by the meat product 20 being conveyed and moved during the operation of the conveying device 10. That is, during the operation of the conveying device 10, the meat product 20 is on the conveying channel 110 and moves along the conveying direction D. The length direction of the conveying channel 110 may correspond to the conveying direction D, while the width direction of the conveying channel 110 may correspond to a transverse direction L. For example, in case the meat product 20 is placed transversely (as shown in the placement of the meat product in Figures 1 and 7) during the process of being conveyed, the width of the conveying channel 110 may be slightly larger than the overall length of the meat product 20, as shown in Figure 1.

As an example, the meat product 20 may include pork, lamb, etc. However, the present disclosure is not limited thereto and may include any meat product suitable for being processed in slaughterhouse production lines.

The meat inspection system 1000 further includes a ray source 30. As shown in Figure 1, the ray source 30 emits a ray beam R toward the conveying channel 110 to perform ray imaging on the meat product 20. According to an embodiment of the disclosure, the ray beam R is configured to cross the conveying channel 110 along the transverse direction L relative to the conveying direction D. For example, the ray source 30 may emit X-rays, infrared light, or visible light. However, the embodiments of the disclosure are not limited thereto and may include any suitable type of ray sources. In the following description, an X-ray source is taken as an example.

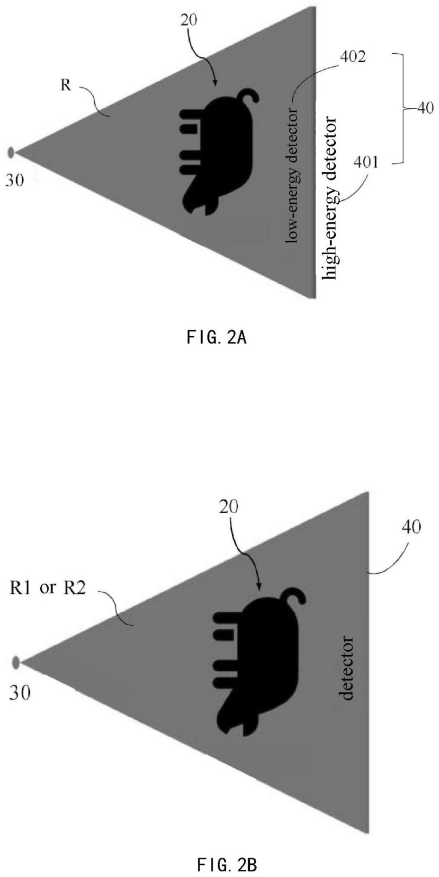

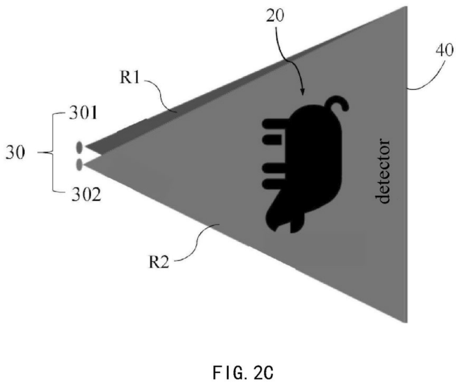

Figures 2A, 2B, and 2C respectively show schematic diagrams of different embodiments of the meat inspection system irradiating a meat product along line C-C in Figure 1. Specifically, Figures 2A, 2B, and 2C respectively show different types of ray sources 30 and detectors 40 that may be applied to the meat inspection system 1000 according to the disclosure.

In the embodiment of Figure 2A, the ray source 30 is configured to emit a single ray beam R, and the detector 40 may be configured to include multiple energy detectors to respectively receive different energy components (e.g., corresponding to different energy ranges of the ray beam R) of the single ray beam R, thereby generating multiple detection signals corresponding to different energy components. For example, Figure 2A shows the detector 40 including two energy detectors, namely a first energy detector and a second energy detector, hereinafter also referred to as a high-energy detector 401 and a low-energy detector 402. However, the embodiments of the present disclosure are not limited thereto and may include more energy detectors as needed.

As shown in Figure 2A, the ray source 30 emits a single ray beam R, and the detector 40 is configured to include the high-energy detector 401 and the low-energy detector 402 to respectively receive a first energy component (i.e., high-energy component) and a second energy component (i.e., low-energy component) of the single ray beam R. For example, the different energy components of the single ray beam R may correspond to radiation of different energy ranges. Thus, the high-energy detector 401 may generate a first detection signal (hereinafter referred to as a high-energy detection signal), while the low-energy detector 402 may generate a second detection signal (hereinafter referred to as a low-energy detection signal) different from the first detection signal.

In the embodiments of Figures 2B-2C, the ray source 30 may be configured to emit multiple energy ray beams with different energy ranges, and the detector 40 may be provided as a single detector to receive the emitted multiple energy ray beams, thereby generating multiple detection signals. For example, Figures 2B-2C show the ray source 30 emitting two energy ray beams, namely a first energy ray beam and a second energy ray beam, hereinafter also referred to as a high-energy ray beam R1 and a low-energy ray beam R2. However, the embodiments of the present disclosure are not limited thereto and the ray source 30 may emit more energy ray beams as needed.

As shown in Figure 2B, the ray source 30 is configured to alternately emit a high-energy ray beam R1 and a low-energy ray beam R2, thereby emitting two energy ray beams. That is, the ray source 30 may be of a type capable of alternately emitting the high-energy ray beam R1 and the low-energy ray beam R2. In a meat inspection process according to an embodiment, when the meat product 20 moves under the ray source 30, the ray source 30 may emit the high-energy ray beam R1 toward the meat product 20 at a first time point and emit the low-energy ray beam R2 toward the meat product 20 at a second time point, thus performing an irradiation process for the meat product 20 that has passed through. As shown in Figure 2B, the emitted high-energy ray beam R1 and low-energy ray beam R2 may be received by the detector 40, thereby generating a high-energy detection signal corresponding to the high-energy ray beam R1 and a low-energy detection signal corresponding to the low-energy ray beam R2 respectively.

As shown in Figure 2C, the ray source 30 is configured to include a first energy ray source 301 (hereinafter also referred to as a high-energy ray source 301) and a second energy ray source 302 (hereinafter also referred to as a low-energy ray source 302). Here, the high-energy ray source 301 emits the high-energy ray beam R1, and the low-energy ray source 302 emits the low-energy ray beam R2, thereby emitting two energy ray beams. In this embodiment, for example, the ray source 30 may be configured in a form of a ray source group. Specifically, the ray source 30 may be a ray source group composed of multiple sub-ray sources, each sub-ray source emitting ray beams of different energy ranges. In the embodiment shown in Figure 2C, the multiple sub-ray sources may be the high-energy ray source 301 and the low-energy ray source 302, thereby enabling the ray source 30 to emit the high-energy ray beam R1 and the low-energy ray beam R2 with different energy ranges. Similarly, the emitted high-energy ray beam R1 and low-energy ray beam R2 may be received by the detector 40, thereby generating a high-energy detection signal corresponding to the high-energy ray beam R1 and a low-energy detection signal corresponding to the low-energy ray beam R2 respectively.

It should be understood that the terms "high-energy" and "low-energy" herein are relative, meaning that the high-energy is different from the low-energy.

According to an embodiment of the disclosure, a processor 60 may be configured to generate a first transmission image (hereinafter referred to as a high-energy transmission image) and a second transmission image (hereinafter referred to as a low-energy transmission image) based on the high-energy detection signal and the low-energy detection signal from the detector 40. The high-energy transmission image and the low-energy transmission image are combined to generate a transmission scanning image SMP of the meat product 20. Thereby, a quantitative indicator QID of the meat product 20 can be calculated to achieve grading of the meat product 20.

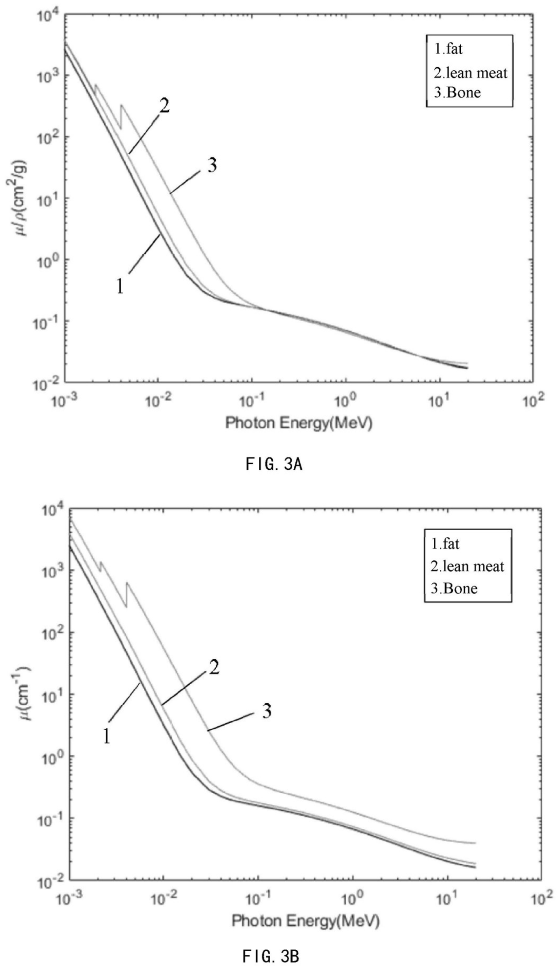

Typically, the meat product 20 mainly includes three types of tissues: fat (adipose tissue), lean meat (muscle tissue), and bone. Taking X-rays as an example, mass attenuation coefficients (µ/ρ) and linear attenuation coefficients (µ) of these three tissues vary with X-ray energy, exhibiting different curves. Figure 3A illustrates curves of the mass attenuation coefficients (µ/ρ) of the three tissues as a function of X-ray energy, and Figure 3B shows curves of the linear attenuation coefficients (µ) of the three tissues as a function of X-ray energy. As shown in Figures 3A-3B, the curves for fat, lean meat, and bone tissues differ from each other. Due to the different attenuation coefficients of these three tissues for X-rays, they can exhibit distinct image features on X-ray images, thereby imaging the distribution of the three tissues in the meat product 20. However, since each tissue has varying thicknesses at different locations, scanning and imaging with a single energy range may yield inaccurate distribution results. For example, a part with thick fat and another part with thin bone may exhibit a same transmittance of the ray beam transmitting these two parts, resulting in identical detection signals and misclassification of these two parts as the same tissue, leading to incorrect tissue distribution results.

Therefore, in an embodiment of the disclosure, the meat inspection system 1000 is configured to perform imaging with at least two energy ranges and generate detection signals corresponding to at least two energy ranges, enabling the same tissue to exhibit different imaging results under radiation of different energy ranges. Since the same tissue exhibits different transmittance under radiation of different energy ranges, analyzing each tissue by combining multiple detection signals can accurately obtain the distribution of the three tissues in the meat product 20, thereby generating a transmission scanning image of the meat product 20. The transmission scanning image can show general contours, image features, and distributions of respective tissues in the corresponding meat product 20, and optionally, different tissues are represented with different grayscales or colors.

In an embodiment, the processor 60 may calculate the quantitative indicator based on the generated transmission scanning image. For example, the processor 60 may calculate the composition ratios and density information of the three tissues in the meat product 20 based on their distribution in the transmission scanning image, thereby calculating a lean meat percentage of the entire meat product 20 as the quantitative indicator. For example, the lean meat percentage may be defined as: lean meat mass/total mass. However, the quantitative indicator is not limited to this embodiment of the present disclosure.

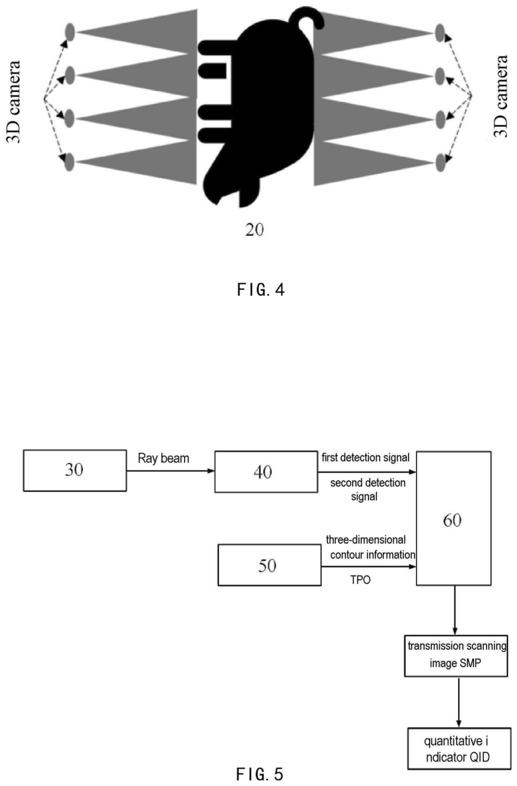

In another embodiment, as shown in Figures 4-5, the meat inspection system 1000 may further include a three-dimensional imaging device 50. The three-dimensional imaging device 50 is configured to obtain three-dimensional contour information TPO of the meat product 20. The processor 60 may calculate the quantitative indicator QID by further combining the three-dimensional contour information TPO based on the high-energy transmission image and the low-energy transmission image. For example, the three-dimensional imaging device 50 may include at least one of a 3D camera, a visible light camera, an infrared camera, or a LiDAR camera.

Figure 4 shows a schematic diagram of a three-dimensional imaging device for a meat product according to an embodiment of the present disclosure. In Figure 4, a 3D camera is shown as an example. To assist the ray imaging system in obtaining more accurate quantitative indicator calculations, the present disclosure employs an auxiliary means (i.e., the three-dimensional imaging device 50) to obtain the three-dimensional contour information TPO of the meat product, i.e., the relative coordinate position of the surface of the meat product in three-dimensional space. For example, after generating the high-energy transmission image and the low-energy transmission image based on the received high-energy detection signal and low-energy detection signal, the processor 60 may further refer to the three-dimensional contour information TPO obtained from the three-dimensional imaging device 50. By combining the positional parameters and width information in the three-dimensional contour information TPO, the distribution of the three tissues can be more accurately distinguished and divided, thereby generating the transmission scanning image SMP. This can improve the calculation accuracy of the processor 60 and reduce errors in calculating the quantitative indicator QID. As another example, the processor 60 may firstly generate the transmission scanning image SMP based on the high-energy transmission image and the low-energy transmission image. Then, the transmission scanning image SMP and the three-dimensional contour information TPO of the meat product 20 are combined to calculate the quantitative indicator QID (e.g., lean meat percentage). By further considering the three-dimensional contour information TPO on the basis of the transmission scanning image SMP, more accurate quantitative indicators can be obtained, enhancing the reliability of the grading results.

Additionally, the three-dimensional contour information TPO may also include local part information of the meat product 20, facilitating the division of the meat product 20 into parts. For example, the meat product 20 may include a front segment, a middle segment, and a rear segment. In this way, based on the tissue distribution information obtained by the processor 60, the tissue distribution in each local part can also be obtained, allowing the quantitative indicator QID (e.g., lean meat percentage) to be calculated separately for each local part. Optionally, specific parts may be graded individually.

Furthermore, in addition to the overall or local lean meat percentage mentioned above, by combining the transmission scanning image SMP and the three-dimensional contour information TPO, other quantitative indicators QID of the meat product 20 can also be calculated, such as the overall length, local part lengths (e.g., the length of the middle segment), local part widths (e.g., the width of the middle segment or the hip), bone lengths in local parts, overall or local part weights, etc., thereby grading the meat product 20. In an embodiment, other information such as the origin, age, and breed of the meat product 20 may also be integrated for comprehensive evaluation and grading. Further, the system 1000 according to the embodiment of the present disclosure may also use X-ray images to detect foreign objects in the meat product 20. For example, if foreign objects are present in the meat product 20, the generated transmission scanning image SMP can directly display them. Therefore, the presence of foreign objects and their sizes can also serve as quantitative indicators for grading or may assist in cutting the meat product 20.

Figure 5 illustrates a data processing block diagram of the meat inspection system 1000 according to an embodiment of the present disclosure. As shown in Figure 5, the processor 60 receives two or more detection signals (e.g., the high-energy detection signal and the low-energy detection signal) from the detector 40 and receives the three-dimensional contour information TPO from the three-dimensional imaging device 50. Thereafter, the processor 60 may process the two or more detection signals and the three-dimensional contour information TPO in combination to accurately determine the distribution of different tissues, thereby calculating the quantitative indicator QID. In this manner, the quantitative indicators of the meat product 20 can be obtained based on ray imaging. By combining the auxiliary information (i.e. the three-dimensional contour information TPO) obtained by the three-dimensional imaging device 50, it is possible to quickly and accurately distinguish different tissues in meat product 20 and calculate quantitative indicators accordingly, thereby achieving meat grading, reducing customer economic losses, and improving customer satisfaction.

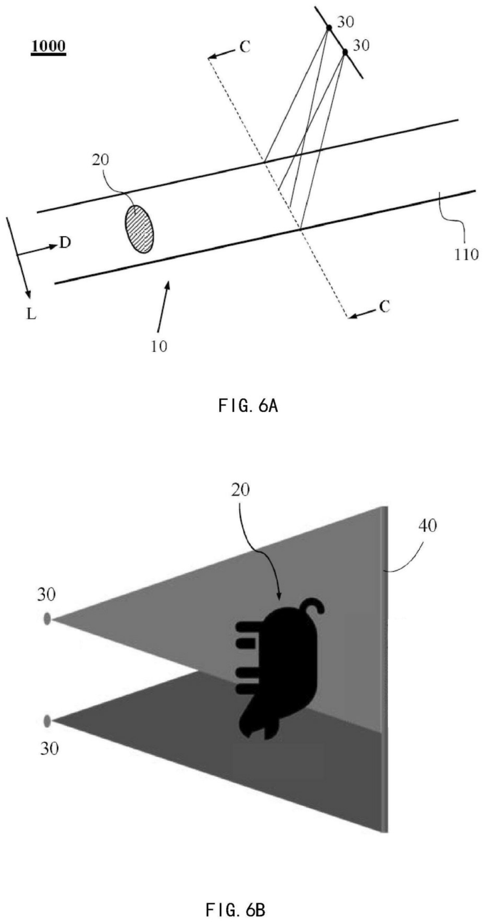

Figure 6A shows a schematic diagram of the meat inspection system according to another embodiment of the present disclosure. Figure 6B shows a schematic diagram of the meat inspection system irradiating a meat product along line C-C in Figure 6A.

In an embodiment, the meat inspection system 1000 may be configured to include one or more ray sources 30 arranged along the transverse direction L relative to the conveying direction D, such that the ray beams sequentially emitted from one side of the conveying channel 110 to the other side cover the transverse width of the conveying channel 110.

Generally, to obtain an X-ray image of the entire meat product for calculating quantitative indicators such as the lean meat percentage, single-scanning or multi-scanning imaging modes may be utilized. Single-scanning refers to completing the scanning of the entire meat product by performing single-pass scanning to directly obtain the image of the entire meat product, while multi-scanning involves obtaining scanned images of local regions of the meat product separately and then stitching them together to indirectly obtain a complete image of the meat product. The system for the single-scanning has the advantage of obtaining a complete image of the meat product in a single-pass scanning, with a shorter scanning time, ensuring the throughput of the meat inspection production line. However, since meat products are typically large in size, the X-ray incident angles in the edge regions of the X-ray imaging area may be larger, potentially causing issues such as difficulty in X-ray penetration due to increased thickness. Additionally, geometric distortions caused by oblique incidence may lead to larger errors in calculating quantitative indicators such as lean meat percentage (under ideal conditions, X-ray beams should perpendicularly irradiate onto the meat product for minimal calculation errors). In light of the potential issues of excessive incident angles in single-scanning imaging, this disclosure proposes a multi-scanning imaging mode.

As an example, Figures 2A-2C show an arrangement of the meat inspection system 1000 including one ray source 30. As another example, Figures 6A-6B show an arrangement of the meat inspection system 1000 including two ray sources 30. As shown in Figures 6A-6B, the meat inspection system 1000 includes two ray sources 30. The two ray sources 30 are arranged along a transverse direction L of the conveying channel 110. That is, an imaginary connecting line between the two ray sources 30 may extend parallel to the transverse direction L. According to an embodiment of the present disclosure, two ray sources are employed, compared to the case of using only one ray source, the divergence angle of the X-ray beam is reduced, and the incident tilt angle of X-rays in the edge regions of the imaging area is decreased. Two overlapping X-ray images of the meat product 20 can be obtained by two scans, which then are stitched to obtain a complete image of the meat product 20. Additionally, the two ray sources 30 may be configured to sequentially emit ray beams, thereby completing the scanning process to achieve stitched imaging.

As another example, Figure 7 shows the arrangement of a distributed ray source in a meat detection system 1000 according to an embodiment of the present disclosure. As shown in Figure 7, the meat inspection system 1000 is configured to include multiple ray sources 30. That is, the multiple ray sources 30 are arranged as a distributed ray source configuration. In this way, the divergence angle of the X-ray beam is further reduced, approaching a pencil beam, while the incident tilt angle of X-rays in the edge regions of the imaging area is further minimized. This further decreases calculation errors in quantitative indicators and enhances the operational reliability of the meat inspection system 1000. Subsequently, a complete image of the meat product can be obtained by stitching together the results of multiple scans. In the embodiment as shown in Figure 7, the multiple ray sources 30 may be arranged along the transverse direction L of the conveying channel 110, similar to the two ray sources 30 shown in Figure 6A. Additionally, these multiple ray sources sequentially emit ray beams from one side of the conveying channel 110 to the other (e.g., along the direction S) to cover the transverse width of the conveying channel 110, thereby completing the scan. Although Figure 7 exemplifies the detector 40 as a dual-energy detector comprising a high-energy detector and a low-energy detector, the present disclosure is not limited to this, for example, energy-spectrum detectors or multi-energy detectors are included in the detector 40.

In an embodiment, the meat inspection system 1000 according to the present disclosure may also be configured to generate at least one additional detection signal, which differs from the high-energy and low-energy detection signals. In such cases, the processor 60 generates the transmission scan image SMP based on a greater number (e.g., more than two) of detection signals. As described above, according to an embodiment of the present disclosure, the more detection signals are produced, the higher the accuracy and reliability of the generated transmission scan image SMP, and the smaller the error in the calculated quantitative indicators QID.

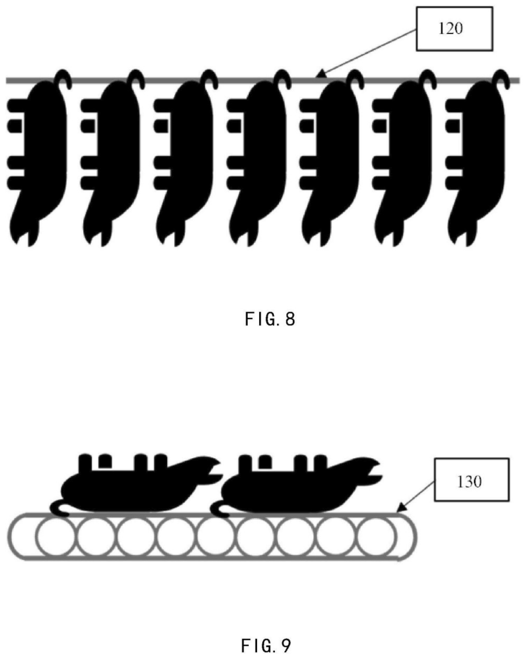

In an embodiment, the conveying device 10 includes an overhead rail device 120. The overhead rail device 120 is configured to move the meat product 20 by suspending it. Figure 8 illustrates the overhead rail device 120 according to an embodiment of the present disclosure. As shown in Figure 8, for example, the meat product 20 may be suspended on the overhead rail via hooks on the overhead rail device 120 and moved along a conveying direction D by means of hinges on the overhead rail device 120, thereby performing suspended scanning inspection.

In another embodiment, the conveying device 10 comprises a conveyor belt 130. The conveyor belt 130 is configured to allow the meat product 20 to be placed thereon for conveying it. Figure 9 illustrates the conveyor belt 130 according to an embodiment of the present disclosure. As shown in Figure 9, for example, the meat product 20 may be laid flat on the conveyor belt 130 and moved along the conveying direction D as the conveyor belt 130 rotates, thereby performing scanning inspection.

Optionally, one or more posture adjustment device may be provided to coordinate with the conveying device 10 in adjusting the posture of meat products 20 to be loaded onto the conveying channel 110. This enables the meat products 20 to enter the scanning area in an optimal posture for achieving a best detection result.

A meat inspection method based on the meat inspection system 1000 according to an embodiment of the present disclosure will now be described with reference to Figures 1 and 5.

As shown in Figures 1 and 5, for example, the meat product 20 is placed on the conveying device 10 so as to be moved along the conveying direction D. The ray source 30 may emit a ray beam toward the conveying channel 110 defined by the conveying device 10, thereby scanning the meat product 20 that moves past the ray source 30. The detector 40 may receive the ray beam emitted by the ray source 30 and generate a first detection signal and a second detection signal respectively. The detector 40 may communicate the first and second detection signals to the processor 60. The processor 60 may generate a first transmission image and a second transmission image based on the first and second detection signals. Optionally, the processor 60 may also obtain three-dimensional contour information TPO of the meat product 20 generated by the three-dimensional imaging device 50. As an example, the three-dimensional imaging device 50 may be arranged near the ray source 30 or near the conveying device 10. However, embodiments of the present disclosure are not limited thereto. Subsequently, the processor 60 may generate a transmission scan image SMP of the meat product 20 based on the first transmission image, the second transmission image, and/or the three-dimensional contour information TPO, from which distribution information and density information of the three types of tissues, among other parameters, may be obtained. Thereby, the processor 60 further calculates a quantitative indicator QID of the meat product 20 and grades the meat product based on the quantitative indicator QID. For example, the quantitative indicator QID may include at least one of the lean meat percentage, length information, width information, weight information, or inclusions.

Advantageously, according to the various embodiments described in the present disclosure, the meat inspection system 1000 and meat inspection method can obtain various quantitative indicators such as lean meat percentage through imaging the meat product, which can guide the grading of meat and improve the accuracy of grading in meat slaughterhouses and processing plants, thereby achieving better economic benefits. The meat inspection system 1000 disclosed herein can be applied to the production line of current slaughterhouses, requiring only a small amount of modification to complete the production process as part of the production line, achieving quantitative and accurate meat grading, and real-time settlement with upstream suppliers. Therefore, it can mitigate or overcome the technical defects in current solutions employing backfat measurement, which only observe local information and are difficult to obtain the overall fat and lean situation of a pig, and require human observation during grading, which heavily relies on the experience and personal qualities of the observer. It also overcomes the technical problems of expensive equipment, limited detection results due to databases, and difficulty in matching complex domestic pig sources related to ultrasonic inspection methods.

It should be understood that various procedures of method shown above can be reordered, or some steps can be added or deleted. For example, the steps described in the present disclosure can be executed in parallel, sequentially, or in different orders, as long as they can achieve the desired results of the technical solution of the present disclosure, which will not be limited.

The above specific embodiments do not constitute limitations on the scope of the present application. Those skilled in the art should understand that various modifications, combinations, sub combinations, and substitutions can be made to them based on design requirements and other factors. Any modifications, equivalent substitutions, and improvements made within the spirit and principles of the disclosure shall be encompassed within the scope of this application.

## Claims

1. A meat inspection system, comprising:
a conveying device defining a conveying channel for conveying a meat product, wherein the meat product moves along a conveying direction on the conveying channel;
a ray source configured to emit a ray beam toward the conveying channel, the ray beam crossing the conveying channel along a transverse direction relative to the conveying direction;
a detector configured to receive the ray beam to generate a first detection signal and a second detection signal respectively, wherein the first detection signal and the second detection signal correspond to radiations of different energy ranges; and
a processor configured to generate a first transmission image and a second transmission image based on the first detection signal and the second detection signal, generate a transmission scan image of the meat product by combining the first transmission image and the second transmission image, and calculate a quantitative indicator of the meat product based on the transmission scan image to grade the meat product.

2. The meat inspection system according to Claim 1, further comprising a three-dimensional imaging device configured to obtain three-dimensional contour information of the meat product,
wherein the processor calculates the quantitative indicator based on the three-dimensional contour information, the first transmission image and the second transmission image.

3. The meat inspection system according to Claim 1, wherein the ray beam emitted from the ray source is a single ray beam, and the detector is configured to include a first energy detector and a second energy detector, wherein the first energy detector receives a first energy component of the single ray beam to generate the first detection signal, and the second energy detector receives a second energy component of the single ray beam to generate the second detection signal.

4. The meat inspection system according to Claim 1, wherein the ray beam emitted from the ray source includes a first energy ray beam and a second energy ray beam with different energy ranges, and the detector is configured to receive the first energy ray beam and the second energy ray beam to generate the first detection signal and the second detection signal, respectively.

5. The meat inspection system according to Claim 4, wherein the ray source is configured to alternately emit the first energy ray beam and the second energy ray beam.

6. The meat inspection system according to Claim 4, wherein the ray source is configured to include a first energy ray source and a second energy ray source, wherein the first energy ray source emits the first energy ray beam, and the second energy ray source emits the second energy ray beam.

7. The meat inspection system according to Claim 1, wherein the system is configured to include one or multiple ray sources, the multiple ray sources are arranged along the transverse direction, such that ray beams sequentially emitted from one side of the conveying channel to the other side of the conveying channel cover a transverse width of the conveying channel.

8. The meat inspection system according to Claim 1, wherein the detector is further configured to generate at least one additional detection signal, wherein the additional detection signal is different from the first detection signal and the second detection signal.

9. The meat inspection system according to Claim 8, wherein an energy range corresponding to the additional detection signal is different from energy ranges corresponding to the first detection signal and the second detection signal.

10. The meat inspection system according to Claim 2, wherein the processor is further configured to classify parts of the meat product based on the three-dimensional contour information and calculate quantitative indicators for different parts of the meat product based on the transmission scanning image, thereby grading the different parts of the meat product.

11. The meat inspection system according to Claim 2, wherein the three-dimensional imaging device includes at least one selected from a group consisting of a 3D camera, a visible light camera, an infrared camera and a LiDAR camera.

12. The meat inspection system according to any one of Claims 1-11, wherein the conveying device comprises:
an overhead rail device configured to move the meat product with the meat product being suspended; or
a conveyor belt configured to allow the meat product to be placed thereon for conveying the meat product.

13. The meat inspection system according to Claim 12, wherein the meat inspection system further includes a posture adjustment device to adjust a posture of the meat product to be loaded onto the conveying channel in coordination with the conveying device.

14. The meat inspection system according to any one of Claims 1-13, wherein the quantitative indicator includes at least one selected from a group consisting of lean meat percentage, length information, width information, weight information and inclusions.

15. A meat inspection method, comprising:
conveying a meat product by a conveying device, wherein the conveying device defines a conveying channel for conveying the meat product, and the meat product moves along a conveying direction on the conveying channel;
emitting a ray beam toward the conveying channel by a ray source, wherein the ray beam crosses the conveying channel along a transverse direction relative to the conveying direction;
receiving the ray beam by a detector to generate a first detection signal and a second detection signal respectively, wherein the first detection signal and the second detection signal correspond to radiations of different energy ranges;
generating a first transmission image and a second transmission image by a processor based on the first detection signal and the second detection signal, and generating a transmission scan image of the meat product by combining the first transmission image and the second transmission image, thereby calculating a quantitative indicator of the meat product to grade the meat product.

16. The method according to Claim 15, further comprising:
obtaining three-dimensional contour information of the meat product by a three-dimensional imaging device,
wherein the processor calculates the quantitative indicator based on the three-dimensional contour information, the first transmission image and the second transmission image.

17. The method according to Claim 15, wherein the ray beam emitted from the ray source is a single ray beam, and the detector is configured to include a first energy detector and a second energy detector, wherein the first energy detector receives a first energy component of the single ray beam to generate the first detection signal, and the second energy detector receives a second energy component of the single ray beam to generate the second detection signal.

18. The method according to Claim 15, wherein the ray beam emitted from the ray source includes a first energy ray beam and a second energy ray beam with different energy ranges, and the detector is configured to receive the first energy ray beam and the second energy ray beam to generate the first detection signal and the second detection signal, respectively.

19. The method according to Claim 18, wherein the ray source is configured to alternately emit the first energy ray beam and the second energy ray beam.

20. The method according to Claim 18, wherein the ray source is configured to include a first energy ray source and a second energy ray source, wherein the first energy ray source emits the first energy ray beam, and the second energy ray source emits the second energy ray beam.

21. The method according to Claim 15, wherein the ray beam is emitted by one or multiple ray sources, the multiple ray sources are arranged along the transverse direction, such that ray beams sequentially emitted from one side of the conveying channel to the other side of the conveying channel cover a transverse width of the conveying channel.

22. The method according to Claim 15, wherein the detector is further configured to generate at least one additional detection signal, wherein the additional detection signal is different from the first detection signal and the second detection signal.

23. The method according to Claim 16, wherein the processor classifies parts of the meat product based on the three-dimensional contour information and calculate quantitative indicators for different parts of the meat product based on the transmission scanning image, thereby grading the different parts of the meat product.

24. The method according to Claim 16, wherein the three-dimensional imaging device includes at least one selected from a group consisting of a 3D camera, a visible light camera, an infrared camera and a LiDAR camera.

25. The method according to any one of Claims 15-24, wherein the conveying device comprises:
an overhead rail device configured to move the meat product with the meat product being suspended; or
a conveyor belt configured to allow the meat product to be placed thereon for conveying the meat product.

26. The method according to Claim 25, further comprising:
adjusting a posture of the meat product to be loaded onto the conveying channel by a posture adjustment device in coordination with the conveying device.

27. The method according to any one of Claims 15-24, wherein the quantitative indicator includes at least one selected from a group consisting of lean meat percentage, length information, width information, weight information and inclusions.
